# FASCICULE DE BREVET EUROPEEN

(11) **EP 3 750 503 B1**
(45) Date de publication et mention de la délivrance du brevet: **05.01.2022**
(21) Numéro de dépôt: 20178745.4
(22) Date de dépôt: 08.06.2020
(51) Int. Cl.: A61B 46/10, A61M 16/00, A61M 16/20

(54) **HOUSSE DE PROTECTION POUR VENTILATEUR MÉDICAL UTILISABLE DANS UN ENVIRONNEMENT CONTAMINÉ DE TYPE NRBC**
SCHUTZHÜLLE FÜR MEDIZINISCHES BEATMUNGSGERÄT, DIE IN EINEM CBRN VERSEUCHTEN UMFELD VERWENDET WERDEN KANN
PROTECTIVE COVER FOR MEDICAL VENTILATOR WHICH CAN BE USED IN AN NRBC TYPE CONTAMINATED ENVIRONMENT

(30) Priorité: 14.06.2019 FR 1906359
(43) Date de publication de la demande: 16.12.2020
(73) Titulaire: Air Liquide Medical Systems, 92160 Antony (FR)
(72) Inventeur: DAVOINE, Romain, 92160 Antony (FR)
(74) Mandataire: Air Liquide

(56) Documents cités:
- FR-A3- 3 034 677
- US-A- 5 458 132
- US-A1- 2012 080 040

## Description

L'invention concerne une housse de protection et un ventilateur médical équipé d'une telle housse de protection utilisable pour traiter en urgence un patient en insuffisance respiratoire dans un environnement contaminé de type NRBC, notamment en cas de conflit, d'attentat ou d'accident industriel.

Dans un hôpital ou sur le terrain, le traitement en urgence d'un patient en insuffisance respiratoire peut s'effectuer à l'aide d'un appareil d'assistance respiratoire, couramment appelé ventilateur médical, fournissant de l'air ambiant, éventuellement enrichi en oxygène provenant d'une bouteille de gaz ou analogue.

Ainsi, FR-A-3034677 propose un ventilateur médical destiné à fournir un gaz respiratoire à un patient souffrant d'insuffisances respiratoires.

Idéalement, un ventilateur médical doit pouvoir être utilisé quels que soient la situation et/ou le lieu dans lequel se trouve le patient, y compris en environnement contaminé de type nucléaire, radiologique, biologique et/ou chimique ou NRBC, par exemple en cas de conflit, d'attentat ou d'accident industriel, c'est-à-dire pour des interventions/utilisations civiles et/ou militaires.

En environnement contaminé de type NRBC, l'air ambiant est contaminé et doit donc être purifié, typiquement filtré, préalablement à son administration au patient au moyen du ventilateur médical.

Or, en pratique, un ventilateur médical ayant été utilisé en environnement NRBC est généralement mis au rebut et détruit ensuite car il est très difficile, voire impossible, de le désinfecter ou de le décontaminer totalement et/ou efficacement du fait notamment des particules contaminées, par exemple des produits chimiques, biologique ou radioactifs pouvant s'immiscer dans les moindres recoins du ventilateur.

Sachant qu'il est par ailleurs souvent obligatoire d'utiliser plusieurs ventilateurs différents entre les interventions et/ou changements de patients traités dans ces environnements contaminés, en particulier de type NRBC, on comprend aisément que la mise au rebut de ces appareils n'est pas satisfaisante car engendre un « gaspillage » de ventilateurs contaminés, sachant par ailleurs que ceux-ci fonctionnent encore parfaitement.

Par ailleurs, US-A-5458132 enseigne un système d'endoscopie dont une partie de l'endoscope manuel est recouvert d'une enveloppe protectrice et US-A-2012/080040 propose un champ opératoire stérile configuré pour isoler une partie d'un dispositif chirurgical au sein d'un environnement stérile.

Le problème qui se pose est donc de pouvoir assister médicalement un patient en insuffisance respiratoire se trouvant dans un environnement contaminé, en particulier de type NRBC et ce, sans engendrer de mise au rebut systématique du ventilateur médical utilisé à cette fin, et en permettant même sa réutilisation ultérieure.

La solution de l'invention est une housse de protection pour ventilateur médical, i.e. appareil d'assistance respiratoire, adaptée à une utilisation en environnement contaminé, en particulier NRBC comprenant une enveloppe périphérique définissant un compartiment interne dimensionné pour recevoir un ventilateur médical, dans laquelle :
- l'enveloppe périphérique comprend plusieurs interfaces de raccordement fixées de manière étanche à ladite enveloppe périphérique, comprenant :
   ▪ une première interface de raccordement permettant le raccordement fluidique d'un circuit patient, et
   ▪ une deuxième interface de raccordement permettant le raccordement fluidique d'un dispositif de purification d'air, typiquement un dispositif de filtration,
- et chaque interface de raccordement comprend au moins un passage interne de gaz avec au moins une entrée de gaz et au moins une sortie de gaz,
caractérisée en ce qu'au moins un dispositif d'obturation est agencé dans le passage interne de gaz d'au moins une interface de raccordement pour normalement obturer ledit passage interne de gaz, c'est-à-dire en l'absence de tout raccordement à l'interface de raccordement considérée.

Selon le mode de réalisation considéré, la housse de protection pour ventilateur médical de l'invention peut comprendre l'une ou plusieurs des caractéristiques suivantes :
- de préférence un dispositif d'obturation est agencé dans chaque passage interne des interfaces de raccordement.
- au moins un dispositif d'obturation agencé dans au moins un passage interne de gaz est un clapet anti-retour.
- chaque clapet anti-retour est normalement fermé pour empêcher de l'air contaminé de s'infiltrer dans le passage interne de gaz dans lequel ledit clapet anti-retour est agencé. Le (les) clapet(s) n'est ouvert que lorsque l'entrée et/ou la sortie de (des) l'interface de raccordement est (sont) connectée(s)/raccordée(s).
- selon un autre mode de réalisation, un bouchon ou couvercle amovible obture, de façon étanche, l'entrée du passage interne de gaz d'au moins une interface de raccordement.
- au moins une partie de l'enveloppe périphérique est formée d'au moins un matériau polymère transparent, de préférence du PVC.
- au moins une partie de l'enveloppe périphérique est souple, de préférence elle comprend une paroi d'enveloppe souple ayant une épaisseur inférieure ou égale à 0,5 mm environ.
- elle comprend une troisième interface de raccordement permettant le raccordement fluidique d'une ligne de contrôle de pression de PEP, c'est-à-dire de pressurisation de PEP (i.e. Pression Expiratoire Positive).
- elle comprend une quatrième interface de raccordement permettant le raccordement fluidique d'une ligne d'alimentation en oxygène, en particulier d'un tuyau flexible alimenté en oxygène fourni par un récipient d'oxygène, telle une bouteille d'oxygène.
- chaque interface de raccordement comprend un corps d'interface traversé par un passage interne de gaz mettant en communication fluidique l'intérieur et l'extérieur de l'enveloppe périphérique.
- chaque interface de raccordement comprend chacune un passage interne de gaz
- les interfaces de raccordement, c'est-à-dire les corps d'interface, sont formées, au moins en partie, d'au moins un matériau semi-rigide.
- ledit au moins un matériau semi-rigide est choisi parmi les polymères thermoplastiques, par exemple de type ABS ou PA, et les élastomères, par exemple de type thermoplastique, polyuréthane ou silicone.
- elle comprend des moyens d'ouverture/fermeture permettant de donner accès au compartiment interne, i.e. volume interne, de la housse afin d'y insérer ou d'en extraire un ventilateur médical.
- les moyens d'ouverture/fermeture comprennent une fermeture à glissière ou "éclair", i.e. « zip », ou analogue, notamment un ou des rubans auto-agrippant de type Velcro^{™}.
- les moyens d'ouverture/fermeture sont conçus pour permettre une fermeture hermétique de la housse.
- elle est étanche, c'est-à-dire que les polluants ne peuvent y pénétrer, lorsque la housse est hermétiquement fermée.
- la surface lisse de la housse est conçue pour repousser les liquides et/ou empêcher l'adhésion de particules solides, par exemple elle peut être recouverte d'un revêtement superficiel adéquat.
- deux des interfaces situées à proximité l'une de l'autre sont dissociées l'une de l'autre, de préférence la deuxième interface et la quatrième interface sont indépendantes l'une de l'autre
- de façon alternative, deux des interfaces situées à proximité l'une de l'autre sont associées, couplées ou agglomérées l'une à l'autre, de préférence la première interface et la troisième interface sont réalisées, solidarisées ou fusionnées en un corps d'interface commun.

L'invention concerne aussi un ensemble de ventilation utilisable en milieu contaminé, en particulier NRBC, comprenant un ventilateur médical, aussi appelé appareil d'assistance respiratoire, autour duquel est agencée de manière étanche, une housse de protection selon l'invention.

Selon le mode de réalisation considéré, l'ensemble de ventilation de l'invention peut comprendre l'une ou plusieurs des caractéristiques suivantes :
- un circuit patient est raccordé à la première interface de raccordement de la housse.
- le circuit patient venant se raccorder à la première interface de raccordement comprend au moins une conduite de gaz, en particulier un tuyau flexible ou analogue.
- le circuit patient comprend au moins une conduite de gaz connectée fluidiquement à une interface respiratoire, en particulier à un masque respiratoire ou analogue.
- un dispositif de purification d'air, de préférence de filtration, est raccordé à la deuxième interface de raccordement de la housse.
- le dispositif de purification d'air comprend une cartouche de filtration conçue et adaptée pour une utilisation en environnement contaminé de type NRBC ou analogue.
- le dispositif de purification d'air comprend une cartouche de filtration configurée pour retenir et/ou éliminer des particules et/ou polluants sous formes solide, liquide et/ou gazeuse.
- le dispositif de purification comprend une cartouche de filtration comprenant au moins un filtre anti-particulaire pour éliminer des particules solides et/ou aérosols, et/ou au moins un filtre à charbon actif ou analogue pour éliminer des polluants gazeux.

L'invention porte aussi sur un ensemble de ventilation utilisable en milieu contaminé, en particulier NRBC comprenant un ventilateur médical autour duquel est agencée de manière étanche, une housse de protection selon l'invention.

La housse de protection selon l'invention est installée autour du ventilateur avant intervention en milieu NRBC, c'est-à-dire dans un lieu non contaminé. La housse assure une protection grâce à son étanchéité, notamment au niveau des entrées et sorties du ventilateur et ce, durant son fonctionnement mais aussi lorsqu'il est à l'arrêt et entre les changements de patients éventuels, ainsi que lors de la décontamination.

Selon le mode de réalisation considéré, l'ensemble de ventilation de l'invention peut comprendre l'une ou plusieurs des caractéristiques suivantes :
- la housse de protection est montée de manière amovible autour du ventilateur médical.
- la housse de protection comprend une enveloppe périphérique comprenant une première, une deuxième et une quatrième interface de raccordement, et éventuellement une troisième interface de raccordement.
- la première interface de raccordement comprend une entrée de gaz raccordée fluidiquement avec une sortie de gaz du ventilateur médical.
- la deuxième interface de raccordement comprend une sortie de gaz libre débouchant dans le compartiment interne de l'enveloppe.
- la quatrième interface de raccordement comprend une sortie d'oxygène raccordée fluidiquement à une entrée d'oxygène du ventilateur médical.
- la troisième interface de raccordement comprend une entrée de gaz raccordée fluidiquement à une sortie de pression de PEP agencée sur un ventilateur médical et/ou une sortie de gaz raccordée fluidiquement à une ligne de contrôle de pression de PEP.
- la première interface de raccordement est raccordée fluidiquement à un circuit patient.
- la deuxième interface de raccordement est raccordée fluidiquement à un dispositif de purification, de préférence de filtration.
- la quatrième interface de raccordement est raccordée fluidiquement à une ligne d'alimentation en oxygène, de préférence une ligne d'alimentation en oxygène alimentée en oxygène par un récipient d'oxygène, en particulier une bouteille d'oxygène.
- le ventilateur médical est un appareil d'assistance respiratoire apte à et conçu pour délivrer du gaz au patient, c'est-à-dire délivrer une ventilation contrôlée à valve permettant d'assurer une ventilation simple à un seul niveau de pression (i.e. CPAP) ou à double niveau de pression (i.e. BiPAP, VSAI), avec contrôle des volumes inspirés et expirés, en fonction des besoins du patient.
- le ventilateur médical comprend une carcasse ou coque externe.
- le ventilateur médical comprend une micro-soufflante motorisée, aussi appelée turbine ou compresseur, permettant d'aspirer de l'air ou de l'air enrichi en oxygène et de le fournir au circuit patient, par exemple un conduit de gaz flexible ou analogue alimentant une interface respiratoire patient, tel un masque respiratoire ou analogue.
- le ventilateur médical comprend une micro-soufflante comprenant un moteur électrique agencé dans un carter périphérique rigide, préférentiellement un moteur sans balai ('brushless' en anglais) conçu pour atteindre une vitesse de rotation jusqu'à 70 000 tr/min ou au-delà.
- la micro-soufflante du ventilateur médical comprend un moteur électrique entraînant en rotation, un arbre rotatif portant une roue à ailettes.
- la micro-soufflante du ventilateur médical comprend un compartiment à roue interne d'une volute surmontant le moteur au sein duquel est agencée la roue à ailettes de manière à y être mobile en rotation.
- Il comprend une électronique de pilotage de la ventilation, par exemple une carte électronique à microprocesseur, et/ou une interface homme-machine ou IHM.

Par ailleurs, l'invention concerne aussi une installation de ventilation comprenant :
- un ensemble de ventilation selon l'invention comprenant un ventilateur médical autour duquel est agencée une housse de protection,
- un circuit patient comprenant un conduit de gaz flexible muni d'une interface respiratoire, ledit conduit de gaz flexible étant relié fluidiquement à la première interface de raccordement de la housse de protection et
- une ligne d'alimentation en oxygène alimentée en oxygène par un récipient d'oxygène, en particulier une bouteille d'oxygène, et reliée fluidiquement à la quatrième interface de raccordement de la housse de protection.

De préférence, l'installation de ventilation comprend aussi une ligne de contrôle de pression de PEP.

L'invention va maintenant être mieux comprise grâce à la description détaillée suivante, faite à titre illustratif mais non limitatif, en référence aux figures annexées parmi lesquelles :
- Fig. 1 représente un ventilateur médical utilisable pour traiter en urgence un patient en insuffisance respiratoire,
- Fig. 2 représente le ventilateur médical de Fig. 1 équipé d'une housse de protection souple selon l'invention, et
- Fig. 3 schématise une interface de raccordement agencée dans la housse de protection souple selon l'invention.

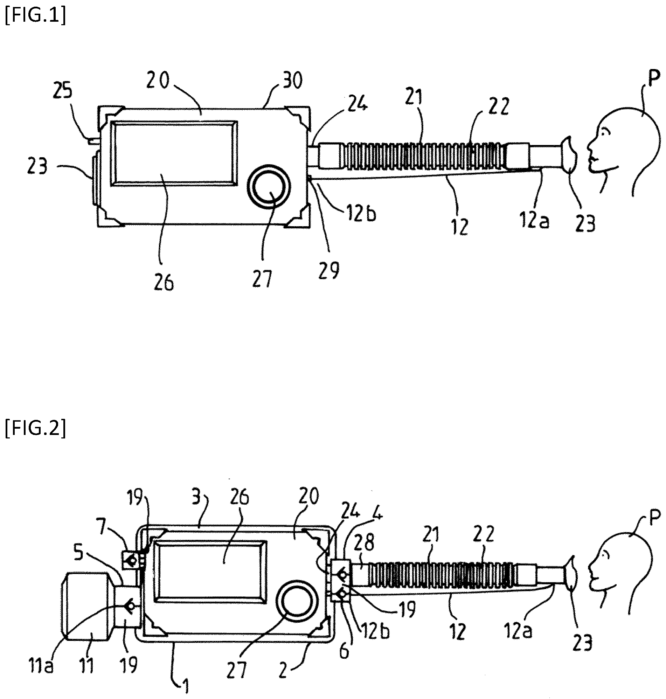

Fig. 1 représente un ventilateur médical 20 utilisable pour traiter en urgence un patient P en insuffisance respiratoire, par exemple un appareil délivrant du gaz à un ou plusieurs niveaux de pression, c'est-à-dire de type CPAP, BPAP, VSAI, CPV ou autre...

Il comprend une carcasse ou coque 30 externe contenant les différents composants permettant d'assurer un bon fonctionnement du ventilateur médical 20. Ainsi, la carcasse 30 du ventilateur médical 20 contient une micro-soufflante motorisée, aussi appelée turbine ou compresseur, permettant d'aspirer de l'air ou de l'air enrichi en oxygène et de le fournir au circuit patient 21, par exemple un conduit de gaz flexible 22 ou analogue alimentant une interface respiratoire patient 23, tel un masque respiratoire ou analogue. Le conduit de gaz 22 flexible du circuit patient 21 vient se raccorder fluidiquement à un connecteur de sortie de gaz 24 porté par la carcasse 30 du ventilateur 20, par vissage, emboitement ou autre.

La micro-soufflante motorisée comprend un moteur électrique agencé dans un carter périphérique rigide servant à protéger le moteur, c'est-à-dire typiquement le stator et le rotor du moteur. Le moteur électrique est préférentiellement sans balai ('brushless' en anglais) et/ou est conçu pour atteindre une vitesse de rotation typiquement de l'ordre de 30 000 à 40 000 tr/min, voire jusqu'à 70 000 tr/min ou même au-delà. Pendant son fonctionnement, le moteur de la micro-soufflante entraine en rotation, un arbre rotatif, ainsi appelé 'axe', portant une roue à ailettes, aussi appelée 'roue à pales'. Cette roue à ailettes a de préférence une section circulaire de diamètre compris entre 20 et 80 mm, typiquement entre 30 et 60 mm. La roue à ailettes est agencée de manière à être mobile en rotation au sein du compartiment à roue interne d'une volute surmontant le moteur électrique et le carter, de manière à générer le flux gazeux à une pression supérieure à la pression atmosphérique (> 1 atm) qui est envoyé au patient P.

La volute peut être formée de deux demi-volutes, à savoir une demi-volute inférieure et une demi-volute supérieure assemblées et fixées de manière solidaire et étanche l'une à l'autre, par exemple par collage ou autre. Un joint d'étanchéité peut être interposé entre ces demi-volutes. La volute a une section générale circulaire et comprend en outre une entrée centrale de gaz par lequel le gaz est aspiré le compartiment à roue situé dans la volute, lors des rotations de la roue, et une sortie de flux de gaz par lequel le flux de gaz généré dans le compartiment à roue, lors des rotations de la roue, ressort de la micro-soufflante, avant d'être envoyé ensuite vers le circuit patient 21.

La micro-soufflante est connectée fluidiquement au circuit patient 21 via un circuit interne de gaz, à savoir un (ou des) passage de gaz agencé dans la carcasse 30 alimenté par la sortie de flux de gaz de la volute.

L'aspiration de l'air par la micro-soufflante se fait via une ouverture 23 aménagée dans la carcasse 30 et un passage d'acheminement d'air interne reliant l'ouverture 23 à l'entrée de la volute de la micro-soufflante.

Lorsqu'un mélange d'air et d'oxygène, i.e. air/O₂, doit être fourni au patient, le mélange air/O₂ se fait en général en amont de l'entrée de la volute de la micro-soufflante. L'oxygène entre dans la carcasse 30 via un connecteur d'entrée d'oxygène 25 permettant de réaliser un raccordement fluidique avec un tuyau flexible alimenté par une source d'oxygène, par exemple une bouteille de gaz ou analogue.

La carcasse 30 du ventilateur médical 20 contient également des moyens de pilotage, par exemple une carte électronique à microprocesseur, tel un microcontrôleur, permettant de contrôler notamment le fonctionnement de la micro-soufflante, à savoir des phases d'accélération et de décélération, et des moyens de fourniture de courant électrique, telle une (ou des) batterie rechargeable ou analogue pour fournir du courant électrique aux différents composants du ventilateur 20 ayant besoin de courant électrique pour fonctionner, notamment aux moyens de pilotage et à la micro-soufflante, via une connectique adaptée, tels des câbles ou circuits électriques.

Par ailleurs, le ventilateur médical 20 comprend aussi une interface d'affichage 26, tel un écran ou analogue, permettant d'afficher des informations, des alarmes, des courbes et/ou toute autre information ou donnée utiles à l'utilisateur, typiquement le personnel soignant ou tout autre personne, et/ou une interface homme/machine ou IHM permettant à l'utilisateur d'opérer une mise en route ou un arrêt du ventilateur, des réglages, des changements de mode ventilatoire, des acquittements d'alarme, des validations de choix etc... Ainsi, l'IHM peut comprendre un (ou des) bouton, touche, curseur, pavé numérique, clavier etc..., par exemple un bouton de sélection rotatif 27.

En outre, le ventilateur médical 20 comprend également une ligne de contrôle de pression de PEP 12, tel un petit conduit souple, reliée à proximité du patient P, par exemple à proximité immédiate et en amont de l'interface respiratoire 23. La ligne de contrôle de pression de PEP 12 comprend, à l'une de ses extrémités, une prise de fourniture de pression de PEP et, à son autre extrémité, un connecteur de raccordement 12b pour la raccorder fluidiquement au ventilateur 20, via un raccord de prise de pression 29 en communication fluidique un circuit interne de mesure de pression agencé dans le ventilateur 20 et aboutissant à un capteur de pression. Le raccordement se fait par exemple par vissage, à baïonnette ou autre.

Afin de permettre d'opérer une ventilation assistée d'un patient dans un environnement contaminé, en particulier de type NRBC, avec un tel ventilateur 20 tout en assurant une protection du ventilateur médical 20 et par la suite, une réutilisation de ce ventilateur médical 20, c'est-à-dire d'éviter une décontamination difficile du ventilateur ou pire, de devoir le mettre au rebut, selon la présente invention, il est proposé d'équiper le ventilateur 20 d'une housse de protection 1 particulière, comme illustré en Fig. 2.

Le ventilateur médical 20 devant être équipé de la housse 1 est avantageusement portatif ou portable, c'est-à-dire qu'il a un poids et des dimensions raisonnables et compatibles avec son transport par un utilisateur, par exemple un poids de moins de 6 kg environ, et une largeur de la carcasse 30 entre 25 et 40 cm environ, une profondeur entre 10 et 15 cm environ, et une hauteur entre 20 et 30 cm environ.

Plus précisément, selon l'invention, la housse 1 de protection comprend, comme montré en en Fig. 2, une enveloppe périphérique 2 souple venant entourer le ventilateur médical 20 et définissant un compartiment interne 3, c'est-à-dire un espace ou volume, dimensionné pour recevoir le ventilateur médical 20. Avantageusement, le compartiment interne 3 de la housse 1 a des dimensions supérieures ou égales à celles du ventilateur 20 qui y est agencé, c'est-à-dire schématiquement des dimensions de la carcasse 30 du ventilateur 20.

L'enveloppe périphérique 2 comprend des moyens d'ouverture/fermeture (non montrés) permettant de donner accès au compartiment interne 3 de la housse 1 afin d'y insérer ou d'en extraire le ventilateur médical 1, puis d'assurer une fermeture hermétique de la housse 1, c'est-à-dire de manière étanche lorsque la housse 1 est hermétiquement fermée, de sorte que les polluants ne puissent y pénétrer, y compris en environnement NRBC. Les moyens d'ouverture/fermeture comprennent par exemple une fermeture éclair, i.e. zip, ou analogue, tel un ruban auto-agrippant.

Selon l'invention, l'enveloppe périphérique 2 comprend par ailleurs plusieurs interfaces de raccordement 4, 5, 6, 7 fixées de manière étanche à ladite enveloppe périphérique 2, c'est-à-dire qu'une entrée de polluants n'est rendue possible au niveau des interfaces de raccordement 4, 5, 6, 7, en particulier entre la structure externe des interfaces de raccordement 4, 5, 6, 7 et la paroi de l'enveloppe périphérique 2 de la housse 1. Leur fixation se fait par exemple par collage, thermocollage/thermosoudage ou mécaniquement par serrage de pièces et de joints.

Plus précisément, l'enveloppe périphérique 2 comprend une première, une deuxième, une troisième et une quatrième interface de raccordement 4, 5, 6, 7.

La première interface de raccordement 4 permet le raccordement fluidique du circuit patient 21, typiquement un conduit flexible de gaz 22, alimentant l'interface respiratoire 23 fournissant le gaz au patient P. Le conduit flexible de gaz 22 comprend un connecteur de fixation 28 permettant de solidariser le conduit flexible de gaz 22 à la première interface de raccordement 4, par exemple par vissage, à baïonnette ou autre.

La deuxième interface de raccordement 5 permet le raccordement fluidique d'un dispositif de purification d'air 11, telle une cartouche de filtration conçue et adaptée pour une utilisation en environnement contaminé de type NRBC, laquelle est configurée pour retenir et/ou éliminer des particules et/ou polluants sous formes solide, liquide et/ou gazeuse. Par exemple, la cartouche de filtration comprend un filtre anti-particulaire pour éliminer des particules solides et/ou aérosols, et/ou du charbon actif ou analogue pour éliminer des polluants gazeux. Le dispositif de purification d'air 11 comprend des moyens de connexion 11a, par exemple par vissage, à baïonnette, par emboitement ou autre, permettant de le solidariser de manière étanche à la deuxième interface de raccordement 5.

La troisième interface de raccordement 6 permet le raccordement fluidique de la ligne de contrôle de pression de PEP 12 servant à moduler la pression d'expiration ou PEP du patient P.

Selon le mode de réalisation choisi, deux interfaces situées à proximité l'une de l'autre peuvent être dissociées l'une de l'autre, comme illustré sur la Fig. 2 pour la deuxième interface 5 et la quatrième interface 7 qui sont indépendantes l'une de l'autre ou, à l'inverse, associées, couplées ou agglomérées l'une à l'autre, comme illustré sur la Fig. 2 pour la première interface 4 et la troisième interface 6 qui sont solidarisées ou fusionnées en un corps d'interface 19 commun.

La quatrième interface de raccordement 7 permet le raccordement fluidique d'une ligne d'alimentation en oxygène, par exemple un tuyau flexible alimenté par une source d'oxygène (non montrés), par exemple une bouteille de gaz ou analogue. Le raccordement de la ligne d'alimentation en oxygène se fait, là aussi, via un connecteur adapté, par exemple par vissage, à baïonnette ou autre.

Selon l'invention, les interfaces de raccordement 4, 5, 6, 7 comprennent chacune un passage interne de gaz 14, c'est-à-dire que chaque interface de raccordement 4, 5, 6, 7 comprend un corps d'interface 19 traversé par un passage interne de gaz 14 mettant en communication fluidique l'intérieur 3 et l'extérieur de l'enveloppe périphérique 2 de la housse 1.

Les corps d'interface 19 des interfaces de raccordement 4, 5, 6, 7 sont formés d'un matériau semi-rigide de type polymère thermoplastique, par exemple ABS, PA ou analogue, ou élastomère, par exemple polyuréthane, silicone ou analogue.

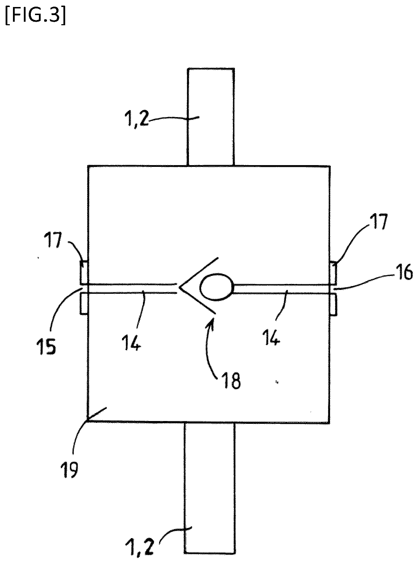

Comme illustré en Fig. 3, les passages internes de gaz 14 traversant les corps d'interface 19 des interfaces de raccordement 4, 5, 6, 7 comprennent chacun une entrée de gaz 15 et au moins une sortie de gaz 16, c'est-à-dire des orifices d'entrée et de sortie de gaz permettant au gaz de pénétrer dans et ensuite de ressortir desdits passages internes de gaz 14, donc de cheminer au travers des interfaces de raccordement 4, 5, 6, 7.

On prévoit par ailleurs au moins un dispositif d'obturation 18 que l'on agence de préférence dans chaque passage interne de gaz 14 pour normalement obturer chaque passage interne de gaz 14 des interfaces de raccordement 4, 5, 6, 7, c'est-à-dire que lorsque les interfaces de raccordement 4, 5, 6, 7 ne sont pas utilisées et qu'aucune ligne d'alimentation en gaz, ligne de contrôle de pression de PEP 12 ou dispositif de purification 11 n'y est raccordé. Les dispositif d'obturation 18 obturent, par défaut, les passage interne de gaz 14 des interfaces de raccordement 4, 5, 6, 7 de manière à empêcher toute circulation de gaz dans ceux-ci, en particulier toute entrée de gaz dans le compartiment interne 3 de l'enveloppe 2 de la housse 1.

Les dispositif d'obturation 18 sont préférentiellement des clapets anti-retour qui sont normalement fermés pour empêcher toute circulation de gaz, notamment pour empêcher de l'air contaminé de s'infiltrer, dans les passages internes de gaz 14 dans lesquels ces clapets anti-retour sont installés. Ils ne sont donc ouverts pour autoriser un passage de gaz que lorsque l'entrée et/ou la sortie des interfaces de raccordement 4, 5, 6, 7 sont connectées ou raccordées fluidiquement à la ligne d'oxygène, au circuit patient 21, la ligne de contrôle de pression de PEP 12 et/ou au dispositif de purification d'air 11.

Selon un autre mode de réalisation, on peut aussi utiliser en lieu et place et/ou en complément, des dispositif d'obturation 18, un ou des bouchons ou couvercles amovibles venant se fixer, de façon étanche, au niveau de l'entrée de gaz des passages internes de gaz 14 d'une ou plusieurs des interfaces de raccordement 4, 5, 6, 7 de manière à les obturer en l'absence de raccordement. De tels bouchons ou couvercles peuvent être utiles lors d'un stockage prolongé et/ou lors d'une désinfection du matériel.

On prévoit aussi sur les corps d'interface 19 des interfaces de raccordement 4, 5, 6, 7 des moyens de raccordement 17, par vissage, emboitement ou autre, permettant d'y raccorder mécaniquement et fluidiquement, d'une part, la ligne d'alimentation en oxygène, le circuit patient 21, la ligne de contrôle de pression de PEP 12 et le dispositif de purification d'air 11 et, d'autre part, le raccord de pression de pression de PEP 29 du ventilateur 20, le connecteur d'entrée d'oxygène 25 et la sortie de gaz 24.

Il est à noter que l'entrée 23 du ventilateur 20 n'est pas obligatoirement reliée à la deuxième interface de raccordement 5 mais peut simplement déboucher dans le compartiment 3 interne de l'enveloppe 2 pour y aspirer l'air qui s'y trouve, lors du fonctionnement de la micro-soufflante.

Par ailleurs, la housse 1 est en partie ou totalement en matériau souple transparent, c'est-à-dire qu'elle est complètement transparente ou bien opaque mais présentes des parties transparentes, telle des fenêtres ou analogue, permettant à un utilisateur de voir le ventilateur 20 qui est agencé dans la housse 1, en particulier de pouvoir lire ou visualiser les informations, données, courbes, alarmes ou autres qui s'affichent sur l'écran d'affichage 26 du ventilateur 20 ou l'IHM 27. Avantageusement, la housse 1 est formée de PVC ou analogue.

Préférentiellement, la housse de protection 1 de l'invention est au minimum IP67 et conçue pour résister à des contraintes environnementales sévères, en particulier à des températures élevées... à l'abrasion et à la déchirure, ainsi qu'aux substances chimiques.

Bien entendu, on peut aussi prévoir des renforts sur la housse, tels des pieds, des éléments « pare-chocs » etc...., en particulier dans sa partie inférieure en contact avec le sol, de sorte d'améliorer sa résistance aux chocs, abrasions, éraflures ou similaires, engendrés par des contacts fréquents avec le sol ou toute autre surface.

De façon générale, un ventilateur médical 20 équipé d'une housse de protection 1 selon l'invention peut être utilisé pour traiter en urgence un ou des patients en insuffisance respiratoire dans un environnement ou milieu contaminé de type NRBC, notamment en cas de conflit, d'attentat ou d'accident industriel.

## Revendications

1. Housse de protection (1) pour ventilateur médical (20) adaptée à une utilisation en environnement contaminé, en particulier NRBC, comprenant une enveloppe périphérique (2) définissant un compartiment interne (3) dimensionné pour recevoir un ventilateur médical (20),dans laquelle :
- l'enveloppe périphérique (2) comprend plusieurs interfaces de raccordement (4, 5, 6, 7) fixées de manière étanche à ladite enveloppe périphérique (2), comprenant :
▪ une première interface de raccordement (4) permettant le raccordement fluidique d'un circuit patient (21), et
▪ une deuxième interface de raccordement (5) permettant le raccordement fluidique d'un dispositif de purification d'air (11),
- et chaque interface de raccordement (4, 5, 6, 7) comprend au moins un passage interne de gaz (14) avec au moins une entrée de gaz (15) et au moins une sortie de gaz (16),
**caractérisée en ce qu'**au moins un dispositif d'obturation (18) est agencé dans le passage interne de gaz (14) d'au moins une interface de raccordement (4, 5, 6, 7) pour normalement obturer ledit passage interne de gaz (14).

2. Housse de protection selon la revendication précédente, **caractérisée en ce qu'**au moins un dispositif d'obturation (18) est agencé dans le passage interne de gaz (14) de chaque interface de raccordement (4, 5, 6, 7).

3. Housse de protection selon l'une des revendications précédentes, **caractérisée en ce qu'**au moins une partie de l'enveloppe périphérique (2) est formée d'au moins un matériau polymère transparent, de préférence un PVC.

4. Housse de protection selon l'une des revendications précédentes, **caractérisée en ce qu'**elle comprend en outre :
- une troisième interface de raccordement (6) permettant le raccordement fluidique d'une ligne de contrôle de pression de PEP (12), et/ou
- une quatrième interface de raccordement (7) permettant le raccordement fluidique d'une ligne d'alimentation en oxygène.

5. Housse de protection selon la revendication 2, **caractérisée en ce qu'**au moins un dispositif d'obturation (18) est un clapet anti-retour.

6. Housse de protection selon l'une des revendications précédentes, **caractérisée en ce que** les interfaces de raccordement (4, 5, 6, 7) sont formées d'au moins un matériau semi-rigide, de préférence ledit au moins un matériau semi-rigide est choisi parmi les polymères thermoplastiques et les élastomères.

7. Ensemble de ventilation (1, 20) utilisable en milieu contaminé, en particulier NRBC comprenant le ventilateur médical (20) autour duquel est agencée de manière étanche, une housse de protection (1) selon l'une des revendications précédentes.

8. Ensemble de ventilation selon la revendication 7, **caractérisé en ce que** la housse de protection (1) comprend l'enveloppe périphérique (2) comprenant la première (4), la deuxième (5) et une quatrième interface (7) de raccordement, où :
- la première interface de raccordement (4) comprend une entrée de gaz (15) raccordée fluidiquement avec une sortie de gaz (24) du ventilateur médical (20),
- la deuxième interface de raccordement (5) comprend une sortie de gaz libre (16) débouchant dans le compartiment interne (3) de l'enveloppe (2), et/ou
- la quatrième interface de raccordement (7) comprend une sortie d'oxygène (16) raccordée fluidiquement à une entrée d'oxygène (25) du ventilateur médical (20).

9. Ensemble de ventilation selon l'une des revendications 7 ou 8, **caractérisé en ce que** :
- la première interface de raccordement (4) est raccordée fluidiquement à un circuit patient (21),
- la deuxième interface de raccordement (5) est raccordée fluidiquement à un dispositif de purification d'air (11), et/ou
- la quatrième interface de raccordement (7) est raccordée fluidiquement à une ligne d'alimentation en oxygène, de préférence une ligne d'alimentation en oxygène alimentée en oxygène par un récipient d'oxygène, en particulier une bouteille d'oxygène, de préférence l'enveloppe périphérique (2) comprend en outre une troisième interface de raccordement (6) comprenant une entrée de gaz (15) raccordée fluidiquement à une sortie de pression de PEP (29) agencée sur un ventilateur médical (20) et/ou une sortie de gaz (16) raccordée fluidiquement à une ligne de contrôle de pression de PEP (12).

10. Installation de ventilation (1, 20, 21) comprenant :
- un ensemble de ventilation selon l'une des revendications 7 à 9,
- un circuit patient (21) comprenant un conduit de gaz flexible (22) muni d'une interface respiratoire (23), ledit conduit de gaz flexible (22) étant relié fluidiquement à la première interface de raccordement (4) de la housse de protection (1) et
- une ligne d'alimentation en oxygène alimentée en oxygène par un récipient d'oxygène, en particulier une bouteille d'oxygène, et reliée fluidiquement à la quatrième interface de raccordement (7) de la housse de protection (1).

## Patentansprüche

1. Schutzhülle (1) für ein medizinisches Beatmungsgerät (20), die für eine Verwendung in einer insbesondere CBRN-kontaminierten Umgebung ausgeführt ist, umfassend eine Umfangsumhüllung (2), die ein inneres Abteil (3) definiert, das zur Aufnahme eines medizinischen Beatmungsgeräts (20) bemessen ist, wobei:
- die Umfangsumhüllung (2) mehrere Anschlussschnittstellen (4, 5, 6, 7) umfasst, die dicht an der Umfangsumhüllung (2) befestigt sind und Folgendes umfassen:
• eine erste Anschlussschnittstelle (4), die den fluidischen Anschluss eines Patientenkreislaufs (21) gestattet, und
• eine zweite Anschlussschnittstelle (5), die den fluidischen Anschluss einer Luftreinigungsvorrichtung (11) gestattet,
- und jede Anschlussschnittstelle (4, 5, 6, 7) mindestens einen inneren Gasdurchgang (14) mit mindestens einem Gaseingang (15) und mindestens einem Gasausgang (16) umfasst,
**dadurch gekennzeichnet, dass** mindestens eine Verschlussvorrichtung (18) in dem inneren Gasdurchgang (14) mindestens einer Anschlussschnittstelle (4, 5, 6, 7) angeordnet ist, um den inneren Gasdurchgang (14) normalerweise zu verschließen.

2. Schutzhülle nach dem vorhergehenden Anspruch, **dadurch gekennzeichnet, dass** mindestens eine Verschlussvorrichtung (18) in dem inneren Gasdurchgang (14) jeder Anschlussschnittstelle (4, 5, 6, 7) angeordnet ist.

3. Schutzhülle nach einem der vorhergehenden Ansprüche, **dadurch gekennzeichnet, dass** mindestens ein Teil der Umfangsumhüllung (2) aus mindestens einem transparenten Polymermaterial, vorzugsweise einem PVC, ausgebildet ist.

4. Schutzhülle nach einem der vorhergehenden Ansprüche, **dadurch gekennzeichnet, dass** sie ferner Folgendes umfasst:
- eine dritte Anschlussschnittstelle (6), die den fluidischen Anschluss einer PEEP-Drucksteuerleitung (12) gestattet, und/oder
- eine vierte Anschlussschnittstelle (7), die den fluidischen Anschluss einer Sauerstoffversorgungsleitung gestattet.

5. Schutzhülle nach Anspruch 2, **dadurch gekennzeichnet, dass** mindestens eine Verschlussvorrichtung (18) ein Rückschlagventil ist.

6. Schutzhülle nach einem der vorhergehenden Ansprüche, **dadurch gekennzeichnet, dass** die Anschlussschnittstellen (4, 5, 6, 7) aus mindestens einem halbstarren Material ausgebildet sind, wobei vorzugsweise das mindestens eine halbstarre Material unter thermoplastischen Polymeren und Elastomeren ausgewählt ist.

7. Beatmungsbaugruppe (1, 20), die in einer insbesondere CBRN-kontaminierten Umgebung verwendbar ist, umfassend das medizinische Beatmungsgerät (20), um das eine Schutzhülle (1) nach einem der vorhergehenden Ansprüche dicht angeordnet ist.

8. Beatmungsbaugruppe nach Anspruch 7, **dadurch gekennzeichnet, dass** die Schutzhülle (1) die Umfangsumhüllung (2) umfasst, die die erste (4), die zweite (5) und eine vierte Anschlussschnittstelle (7) umfasst, wobei:
- die erste Anschlussschnittstelle (4) einen Gaseingang (15) umfasst, der fluidisch an einem Gasausgang (24) des medizinischen Beatmungsgeräts (20) angeschlossen ist,
- die zweite Anschlussschnittstelle (5) einen freien Gasausgang (16) umfasst, der in das innere Abteil (3) der Umhüllung (2) mündet, und/oder
- die vierte Anschlussschnittstelle (7) einen Sauerstoffausgang (16) umfasst, der fluidisch an einem Sauerstoffeingang (25) des medizinischen Beatmungsgeräts (20) angeschlossen ist.

9. Beatmungsbaugruppe nach einem der Ansprüche 7 oder 8, **dadurch gekennzeichnet, dass**:
- die erste Anschlussschnittstelle (4) fluidisch an einem Patientenkreislauf (21) angeschlossen ist,
- die zweite Anschlussschnittstelle (5) fluidisch an einer Luftreinigungsvorrichtung (11) angeschlossen ist und/oder
- die vierte Anschlussschnittstelle (7) fluidisch an einer Sauerstoffversorgungsleitung angeschlossen ist, vorzugsweise einer Sauerstoffversorgungsleitung, die mittels eines Sauerstoffbehälters, insbesondere einer Sauerstoffflasche, mit Sauerstoff versorgt wird, wobei vorzugsweise die Umfangsumhüllung (2) ferner eine dritte Anschlussschnittstelle (6) umfasst, die einen Gaseingang (15) umfasst, der fluidisch an einem PEEP-Druckausgang (29) angeschlossen ist, der an einem medizinischen Beatmungsgerät (20) angeordnet ist, und/oder einen Gasausgang (16), der fluidisch an einer PEEP-Drucksteuerleitung (12) angeschlossen ist.

10. Beatmungsanlage (1, 20, 21), umfassend:
- eine Beatmungsbaugruppe nach einem der Ansprüche 7 bis 9,
- einen Patientenkreislauf (21), der eine flexible Gasleitung (22) umfasst, die mit einer Beatmungsschnittstelle (23) versehen ist, wobei die flexible Gasleitung (22) fluidisch mit der ersten Anschlussschnittstelle (4) der Schutzhülle (1) verbunden ist, und
- eine Sauerstoffversorgungsleitung, die mittels eines Sauerstoffbehälters, insbesondere einer Sauerstoffflasche, mit Sauerstoff versorgt wird und fluidisch mit der vierten Anschlussschnittstelle (7) der Schutzhülle (1) verbunden ist.

## Claims

1. Protective cover (1) for a medical ventilator (20) adapted for use in a contaminated environment, in particular an NRBC environment, comprising a peripheral casing (2) defining an internal compartment (3) designed to accommodate a medical ventilator (20), wherein:
- the peripheral casing (2) comprises a plurality of connection interfaces (4, 5, 6, 7) sealably attached to said peripheral casing (2), comprising:
▪ a first connection interface (4) allowing fluid connection of a patient circuit (21), and
▪ a second connection interface (5) allowing fluid connection of an air purification device (11),
- and each connection interface (4, 5, 6, 7) comprises at least one internal gas passage (14) with at least one gas inlet (15) and at least one gas outlet (16),
**characterized in that** at least one sealing device (18) is arranged in the internal gas passage (14) of at least one connection interface (4, 5, 6, 7) for sealing said internal gas passage (14) normally.

2. Protective cover according to the preceding claim, **characterized in that** at least one sealing device (18) is arranged in the internal gas passage (14) of each connection interface (4, 5, 6, 7).

3. Protective cover according to one of the preceding claims, **characterized in that** at least one portion of the peripheral casing (2) is formed by at least one transparent polymer material, preferably PVC.

4. Protective cover according to one of the preceding claims, **characterized in that** it further comprises:
- a third connection interface (6) allowing fluid connection of a PEP pressure control line (12), and/or
- a fourth connection interface (7) allowing fluid connection of an oxygen supply line.

5. Protective cover according to Claim 2, **characterized in that** at least one sealing device (18) is a non-return valve.

6. Protective cover according to one of the preceding claims, **characterized in that** the connection interfaces (4, 5, 6, 7) are formed by at least one semi-rigid material, preferably said at least one semi-rigid material is selected from thermoplastic polymers and elastomers.

7. Ventilation assembly (1, 20) that can be used in a contaminated environment, in particular an NRBC environment, comprising the medical ventilator (20), around which a protective cover (1) according to one of the preceding claims is sealably arranged.

8. Ventilation assembly according to Claim 7, **characterized in that** the protective cover (1) comprises the peripheral casing (2) comprising the first (4), the second (5) and a fourth (7) connection interface, where:
- the first connection interface (4) comprises a gas inlet (15) fluidly connected to a gas outlet (24) of the medical ventilator (20),
- the second connection interface (5) comprises a free gas outlet (16) leading into the internal compartment (3) of the casing (2), and/or
- the fourth connection interface (7) comprises an oxygen outlet (16) fluidly connected to an oxygen inlet (25) of the medical ventilator (20).

9. Ventilation assembly according to one of Claims 7 or 8, **characterized in that**:
- the first connection interface (4) is fluidly connected to a patient circuit (21),
- the second connection interface (5) is fluidly connected to an air purification device (11), and/or
- the fourth connection interface (7) is fluidly connected to an oxygen supply line, preferably an oxygen supply line supplied with oxygen through an oxygen container, in particular an oxygen bottle, preferably the peripheral casing (2) further comprises a third connection interface (6) comprising a gas inlet (15) fluidly connected to a PEP pressure outlet (29) arranged on a medical ventilator (20) and/or a gas outlet (16) fluidly connected to a PEP pressure control line (12) .

10. Ventilation system (1, 20, 21) comprising:
- a ventilation assembly according to one of Claims 7 to 9,
- a patient circuit (21) comprising a flexible gas hose (22) provided with a breathing interface (23), said flexible gas hose (22) being fluidly connected to the first connection interface (4) of the protective cover (1) and
- an oxygen supply line supplied with oxygen by an oxygen container, in particular an oxygen bottle, and fluidly connected to the fourth connection interface (7) of the protective cover (1).
